# EUROPEAN PATENT SPECIFICATION

(11) **EP 2 522 292 B1**
(45) Date of publication and mention of the grant of the patent: **04.07.2018**
(21) Application number: 12003728.8
(22) Date of filing: 10.05.2012
(51) Int. Cl.: A61B 18/18, G01R 29/08

(54) **Laparoscopic port with microwave rectifier**
Laparoskopischer Anschluss mit Mikrowellengleichrichter
Port laparoscopique avec rectification à micro-ondes

(30) Priority: 10.05.2011 US 201113104752
(43) Date of publication of application: 14.11.2012
(73) Proprietor: Covidien LP, Mansfield, MA 02048 (US)
(72) Inventor: Brannan, Joseph D., Erie, CO 80516 (US)
(74) Representative: Soames, Candida Jane

(56) References cited:
- EP-A1- 2 264 828
- EP-A1- 2 468 359
- US-A1- 2002 075 189

## Description

### BACKGROUND

### 1. Technical Field

The present disclosure relates generally to microwave antennas. More particularly, the present disclosure is directed to radiation detectors for microwave ablation antennas.

### 2. Background of Related Art

Treatment of certain diseases requires destruction of malignant tissue growths (e.g., tumors). It is known that tumor cells denature at elevated temperatures that are slightly lower than temperatures injurious to surrounding healthy cells. Therefore, known treatment methods, such as hyperthermia therapy, heat tumor cells to temperatures above 41° C, while maintaining adjacent healthy cells at lower temperatures to avoid irreversible cell damage. Such methods involve applying electromagnetic radiation to heat tissue and include ablation and coagulation of tissue. In particular, microwave energy is used to coagulate and/or ablate tissue to denature or kill the cancerous cells.

Microwave energy is applied via microwave ablation antennas that penetrate tissue to reach tumors. There are several types of microwave antennas, such as monopole and dipole, in which microwave energy radiates perpendicularly from the axis of the conductor. A monopole antenna includes a single, elongated microwave conductor whereas a dipole antenna includes two conductors. In a dipole antenna, the conductors may be in a coaxial configuration including an inner conductor and an outer conductor separated by a dielectric portion. More specifically, dipole microwave antennas may have a long, thin inner conductor that extends along a longitudinal axis of the antenna and is surrounded by an outer conductor. In certain variations, a portion or portions of the outer conductor may be selectively removed to provide more effective outward radiation of energy. This type of microwave antenna construction is typically referred to as a "leaky waveguide" or "leaky coaxial" antenna.

During microwave ablation, unintended field exposure to healthy tissue may occur due to incorrect device use. For example, damage to healthy tissue may occur if a surgeon inserts the probe to an insufficient depth while performing an ablation, the probe slipping out due to surgeon error or fatigue, or activation of the probe prior to placing the probe in tissue. Also, the repercussions of the unintended field exposure may increase during laparoscopic procedures due to high field intensities as a result of an insufflated abdomen acting as a resonant microwave cavity. Burns to the abdominal wall along device/probe insertion tracks have occurred due to these factors.

EP 2 264 828 A1 discloses several embodiments of microwave ablation systems including a microwave ablation antenna radiation detector.

EP 2 468 359 A1 (published after the priority date of the present document) discloses a system comprising an energy applicator and needle assemblies.

US 2002/0075189 A1 discloses a close-proximity radiation detection device.

### SUMMARY

The invention is defined in claim 1.

A radiation detector disposed on a microwave antenna assembly to receive unintended field exposure in an insufflated abdomen. The radiation detector includes a receiving antenna made up of at least two pieces of metal externally attached to the microwave antenna on the distal end so as to be within the abdomen. The radiation detector is adapted to receive errant microwave energy that resonates in the abdomen. A rectifier is coupled between the two pieces of metal, where the pieces of metal are strips, rings, patches, or other geometric combinations. The rectifier is adapted to rectify at least a portion of the errant microwave energy. A filter is coupled to the rectifier and is adapted to convert the rectified microwave energy into a detection signal. An inflatable stop is located on a distal end of the microwave antenna and the stop is inflated when inserted within the abdomen. The inflated stop prevents inadvertent removal of the microwave antenna.

According to one aspect of the disclosure, a radiation detector disposed on a microwave antenna assembly is disclosed. The radiation detector includes a receiving antenna adapted to receive microwave energy. The receiving antenna is formed from two pieces of metal externally attached to a microwave antenna of the microwave antenna assembly. The radiation detector further includes at least one rectifier coupled between the pieces of metal adapted to rectify at least a portion of the microwave energy and a filter coupled to the at least one rectifier and adapted to convert the rectified microwave energy into a detection signal.

According to another aspect of the present disclosure, a microwave antenna assembly is disclosed. The microwave antenna assembly includes a hub adapted to couple the microwave antenna assembly to a microwave generator and a radiating section coupled to the hub through a feedline. The microwave antenna assembly further includes an inflatable stop surrounding the feedline. The inflatable stop is inflated when the feedline is placed within an abdomen of a patient to prevent the radiating section from inadvertently withdrawing from the abdomen. Additionally, the microwave antenna assembly includes a radiation detector disposed on the microwave antenna assembly near the radiating section. The radiation detector includes a receiving antenna adapted to receive microwave energy. The receiving antenna is formed from two pieces of metal externally attached to the feedline within the abdomen. The receiving antenna further includes at least one rectifier coupled between the pieces of metal adapted to rectify at least a portion of the microwave energy that resonates in the abdomen. Additionally, the receiving antenna includes a filter coupled to the at least one rectifier and adapted to convert the rectified microwave energy into a detection signal.

A method for detecting errant microwave energy is also contemplated by the present disclosure. The method includes the steps of receiving resonant microwave energy from an insulfated abdomen with a receiving antenna and rectifying at least a portion of the microwave energy through at least one rectifier coupled to the receiving antenna. Further the method includes the step of filtering the rectified microwave energy through a filter coupled to the at least one rectifier to convert the rectified microwave energy into a detection signal.

### BRIEF DESCRIPTION OF THE DRAWINGS

The above and other aspects, features, and advantages of the present disclosure will become more apparent in light of the following detailed description when taken in conjunction with the accompanying drawings in which:
- Fig. 1: is a schematic diagram of a microwave ablation system according to an embodiment of the present disclosure;
- Figs. 2A-2C: are schematic diagrams of a microwave detector according to different embodiments of the present disclosure;
- Fig. 3: is a schematic diagram of a linear antenna for use in detecting microwaves according to an embodiment of the present disclosure; and
- Fig. 4: is a flow chart of a method according to the present disclosure.

### DETAILED DESCRIPTION

Particular embodiments of the present disclosure as described herein below with reference to the accompanying drawings. In the following description, well-known functions or constructions are not described in detail to avoid obscuring the present disclosure in unnecessary detail.

The present disclosure provides for a radiation detector disposed on a microwave antenna. Generally, the detector is disposed in a location such that any unintended and/or errant radiation of microwave energy within the abdomen is detected. The radiation detector converts the detected radiation into a detection signal, which is then transmitted to a control system (e.g., microwave generator) to either shut off the power supply and/or alert the user.

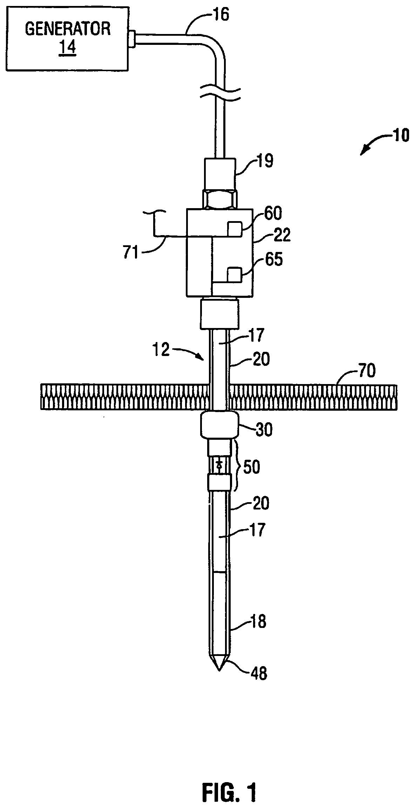

Fig. 1 shows a microwave ablation system 10 that includes a microwave antenna assembly 12 coupled to a microwave generator 14 via a flexible coaxial cable 16. The generator 14 is configured to provide microwave energy at an operational frequency from about 500 MHz to about 10,000 MHz. In the illustrated embodiment, the antenna assembly 12 includes a radiating section 18 connected by feedline 20 (or shaft) to the cable 16. More specifically, the feedline 20 is connected to a hub 22, which is connected to the cable 16 through a cable connector 19. The hub 22 may have a variety of suitable shapes, e.g., cylindrical, rectangular, etc. Further, the antenna assembly 12 includes a radiating section 18 with a tip 48 on the distal end of the feedline 20.

The feedline 20 may be coaxial and include an inner conductor surrounded by an inner insulator, which is, in turn, surrounded by an outer conductor 17 (e.g., a cylindrical conducting sheath). The inner conductor and outer conductor 17 may be constructed of copper, gold, stainless steel or other conductive metals with similar conductivity values. The metals may be plated with other materials, e.g., other conductive materials, to improve their properties, e.g., to improve conductivity or decrease energy loss, etc. In one embodiment, the feedline 20 may be formed from a coaxial, semi-rigid or flexible cable having a wire with a 0.047" outer diameter rated for 50 Ohms.

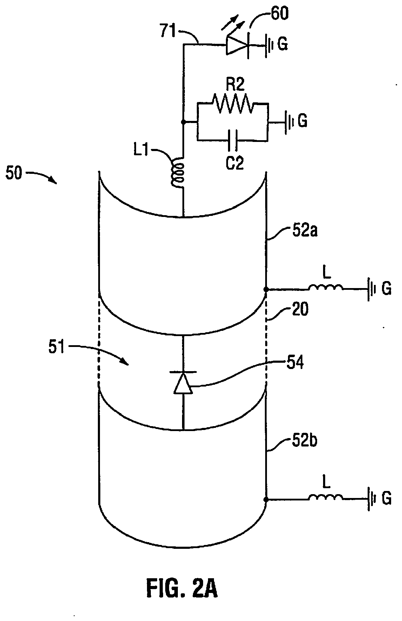

The antenna assembly 12 includes a radiation detector 50 disposed along the feedline 20 just on the inside of the abdominal wall 70. Further, the radiation detector 50 may be located near an inflatable stop 30. The radiation detector 50 is shown in detail in Figs. 2A-2C. The radiation detector 50 is connected through a filter 58 to wire 71. The wire 71 may be disposed anywhere along the antenna assembly 12 such that the wire 71 has minimal effect on the radiation efficiency of the antenna assembly 12. The wire 71 may be connected to a light emitting diode (LED) 60, a speaker 65, and/or a controller (not shown) within the generator 14.

The inflatable stop 30 is a balloon or other inflatable material that surrounds the feedline 20. The stop 30 may be formed from materials having suitable mechanical properties (such as puncture resistance, pin hole resistance, tensile strength, conformability when inflated), chemical properties (such as forming a suitable bond to the feedline 20), and biocompatibility. In another embodiment, the walls of the inflatable stop 30 may be formed from a suitable polyvinyl chloride (PVC). Other suitable materials include polypropylene, polyethylene teraphthalate (PETP), low-density polyethylene (LDPE), silicone, neoprene, polyisoprene, or polyurethane (PU).

The inflatable stop 30 is located on the distal end of the feedline 20 so as to be inside the abdomen wall 70 or body cavity wall. The location of the inflatable stop 30 may be adjusted based on the size of the abdomen and/or the depth necessary to perform the surgery.

Prior to inserting the radiating section 18 and feedline 20 within the patient's abdomen or body cavity, the inflatable stop 30 is in a collapsed form. After inserting the radiating section 18 and feedline 20 within the patient's abdomen, the inflatable stop 30 is inflated using a conduit or catheter (not shown). The inflatable stop 30 may be filled with gaseous or fluid inflation media, e.g., air, water, saline etc., in a selective manner such that inflation media may be introduced and/or withdrawn from inflatable stop 30 as desired. Once inflated, the inflatable stop 30 prevents inadvertent removal of the radiating section 18. Inadvertent removal may cause ablation to the wrong tissue. The inflatable stop 30 is then deflated upon completion of the procedure to allow removal of the radiating section 18 and feedline 20.

Fig. 2A shows a first embodiment of a radiation detector 50. The radiation detector 50 includes two metal rings 52a-52b that wrap around the feed line 20. The metal rings 52a-52b are connected together using a rectifying device 54 across gap 51. The rectifying device 54 may be any type of suitable diodes such as Zener diode, Schottky diode, tunnel diode and the like. One metal ring 52a is connected to LED 60 and ground circuit (R2 and C2 in parallel and G) through inductor L1. LED 60 receives a DC signal from wire 71 and is connected to ground G. Alternatively, LED 60 may be a speaker, and/or a controller within generator 14. Alternatively, both metal rings 52a-52b may be connected to a ground circuit that includes a RF impendence/low DC impedance element L and ground G. The DC ground connections are made in locations of low RF voltage.

The radiation detector 50 may be located anywhere along the feedline 20 and/or radiating section 18 as long as the radiation detector 50 is within the patient's abdomen or body cavity. The radiation detector 50 is typically located along the feedline 20 so as to have gap 51 be in a location of high RF voltage.

The metal rings 52a-52b may be formed from a conformal sheet of conductive material such as copper, gold, stainless steel or other conductive metals with similar conductivity values. The width of each ring may be about 0,10 inches (2,54 mm) to about 2,5 inches (63,5 mm) with a thickness between about 0,001 inches (0,0254 mm) to about 0,010 inches (0,254 mm). The metal rings 52a-52b may be situated over a ground plane with a dielectric insulation providing separation. The dielectric insulation R2 may be formed from a non-conductive conformal material such as polyesters, polyimides, polyamides, polyamide-imides, polyetherimides, polyacrylates, polyethylene terephthalate, polyethylene, polypropylene, polyvinylidene chloride, polysiloxanes, combinations thereof and the like.

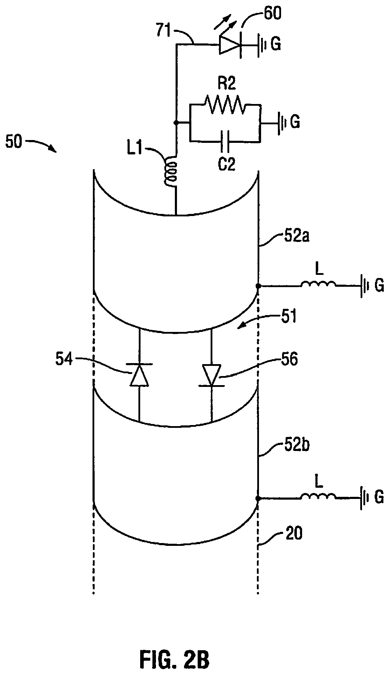

The use of one rectifying device 54 in Fig. 2A allows for half wave rectifying. Fig. 2B shows an alternate embodiment of radiation detector 50 that includes two rectifying devices 54 and 56. The rectifying devices 54 and 56 are soldered to metal pieces 52a-52b in reverse polarity. The rectifying devices 54 and 56 together allow for full wave rectifying.

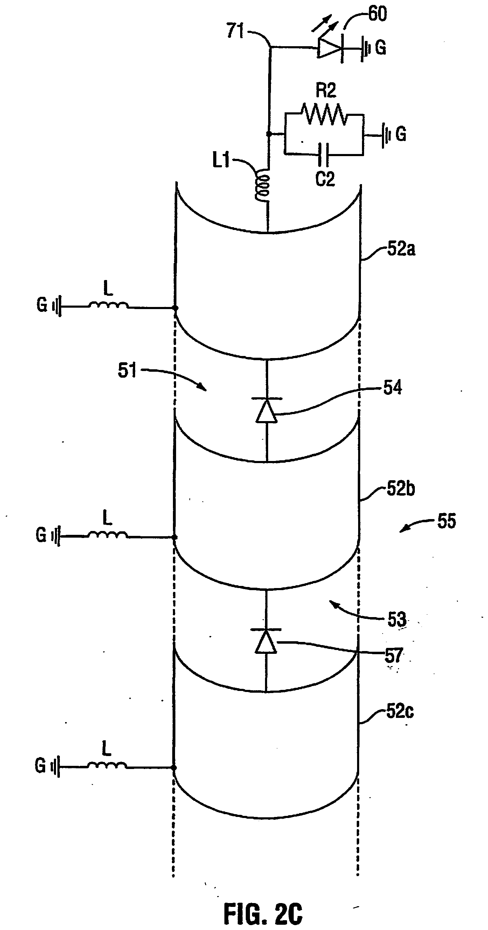

Fig. 2C shows another embodiment of radiation detector 55 that includes three rings 52a-52c. The three rings are connected together with rectifying devices 54 and 57 in the same polarity. The use of three rings allows for a larger antenna aperture and increased bandwidth for detecting rectified microwave energy. Alternatively, the rings 52a-52c may be connected together with the rectifying devices 54 and 57 in reverse polarity. Additionally, two of the rectifying devices (e.g. 54 and 56) may be connected in reverse polarity in each gap 51 and 53, similar to the arrangement shown in Fig. 2B.

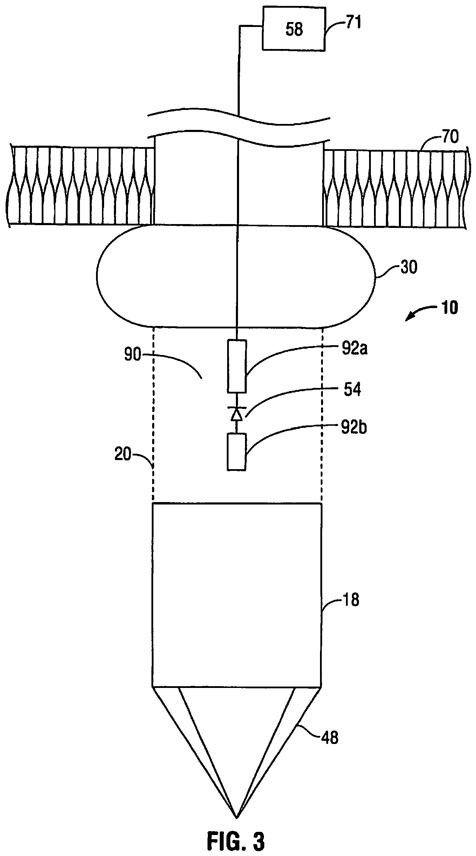

Fig. 3 discloses an alternative radiation detector 90 formed into a linear antenna from two strips of metal 92a-92b. Depending on the operating frequency of the microwave ablation assembly 12, the two strips 92a-92b may have a length and width that range between about 0,1 inches (2,54 mm) and about 2 inches (50,8 mm) with a thickness of about 0,005 inches (0,127 mm). The strips of metal 92a-92b may be connected together with rectifying device 54. Alternatively, the strips of metal 92a-92b may be connected together with two rectifying devices (e.g. 54 and 56) in reverse polarity to allow for full wave rectification, similar to the arrangement shown in Fig. 2B. The rectifying device 54 is connected to filter 58 to convert the signal into a DC signal. The DC signal is sent over wire 71 to a LED 60, speaker 65, and/or a controller in the generator 14. If the DC signal is above a set limit, then the user is notified visually through LED 50, and/or audibly through speaker 65. Alternatively, if the DC signal is above a set limit, then controller may automatically stop sending an electrical signal to the antenna assembly. This limit may vary between about 0.5 volts and about 3 volts based on the operating frequency of the microwave ablation assembly 12. The linear antenna radiation detector 90 may also be used as a patch rectifier or a dipole rectifier.

The strips of metal 92a-92b may be attached to a grounding plane (not shown) with an insulating dielectric between. The strips of metal 92a-92b and grounding plane may be made of conductive material such as copper, gold, stainless steel or other conductive metals with similar conductivity values. The insulating dielectric may be a non-conductive conformal material such as polyesters, polyimides, polyamides, polyamide-imides, polyetherimides, polyacrylates, polyethylene terephthalate, polyethylene, polypropylene, polyvinylidene chloride, polysiloxanes, combinations thereof and the like.

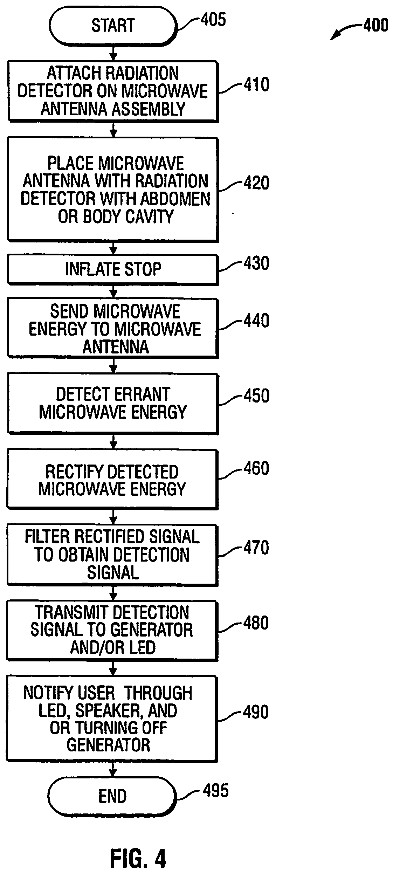

Fig. 4 shows a process 400 for detecting errant microwave energy within an insufflated abdomen with reference to radiation detector 50. It should be appreciated that the method may be practiced with other radiation detectors, such as the radiation detector 90, 55 and the like. The process 400 starts at step 405 with an initial step of attaching the radiating detector 50 on the microwave antenna assembly 12 at step 410 which includes wrapping the metal pieces 52a-52b around feedline 20. The method may also include the step of connecting the radiation detector 50 to the generator 14 or another control system. Next, the microwave antenna 12 with radiation detector 50 is placed within a patient's abdomen or body cavity at step 420. Alternatively, the detector 50 may be placed into the insufflated abdomen via a lap port (not shown) and slid over the radiating section 18 and around feedline 20 after insertion of radiating section 18 and feed line 20 through the abdominal wall to reduce gauge size of the device. Detector 50 interfaces with one or more electrode contacts (not shown) on feedline 20. At step 430, the microwave antenna 12 is secured in place within the abdomen. For example, the inflatable stop 30 may be inflated at step 430. Other types of securement materials may also be contemplated. A controller (not shown) in generator 14 may prevent sending electrical energy to the microwave antenna 12 until the microwave antenna 12 is secured, e.g., until the inflatable stop 30 is inflated. Next, at step 440, microwave energy is sent from the generator 14 to microwave antenna 12.

During operation, any errant microwave radiation outside the desired emission area, such as outside the radiating section 18, is picked up by the radiation detector 50, namely, the metal rings 52a-52b at step 450. The detected microwave energy is then rectified by rectifier 54 at step 460 and the rectified signal is filtered by filter 58 into a detection signal (e.g., a DC voltage signal) at step 470. The detection signal is then transmitted to generator 14, LED 60, and/or speaker 65 at step 480. The generator 14 and/or other control circuitry (not shown) compares the detection signal to a threshold value to determine whether the level of the microwave energy is unsafe. If the determination is made that the level of microwave energy is excessive, the generator 14 may either suspend the supply of microwave energy and/or notify the user of this occurrence at step 490 prior to process 400 ending at step 495. The user may be notified using speaker 65 and/or LED 60. The embodiments aspects and examples which do not fall within the scope of the claims are provided for illustrative purpose only and do not form part of the present invention. The invention is defined in the claims as follows.

## Claims

1. A microwave antenna assembly (12), comprising a microwave antenna, a coaxial feedline (20) and a radiation detector (50, 55), disposed along the coaxial feedline (20) and configured to be positioned inside the abdominal wall; the radiation detector (50; 55) comprising:
a receiving antenna adapted to receive microwave energy, wherein the receiving antenna is formed from two pieces of metal (52a, 52b) externally attached to the microwave antenna;
at least one rectifier (54, 56) coupled between the pieces of metal (52a, 52b) adapted to rectify at least a portion of the microwave energy; and
a filter (58) coupled to the at least one rectifier (54, 56) and adapted to convert the rectified microwave energy into a detection signal,
wherein each of the two pieces of metal (52a, 52b) has a geometry of a ring wrapped around the coaxial feedline (20) and is configured to be inserted into an abdomen or a body cavity, each ring having a width between 2,54 mm (0,10 inches) and 63,5 mm (2,5 inches) and a thickness between 0,0254 mm (0,001 inches) and 0,254 mm (0,010 inches).

2. The microwave antenna assembly according to claim 1, wherein the receiving antenna further comprises:
two dielectric substrates each having a top surface and a bottom surface;
each piece of metal (52a, 52b) is disposed on the top surface of each dielectric substrate; and
a grounding member disposed on the bottom surface of each dielectric substrate, wherein the antenna and grounding members are electrically insulated by the dielectric substrate.

3. The microwave antenna assembly according to claim 2,
wherein the dielectric substrate is formed from a non-conductive, conformal material and the grounding member are formed from a conductive, conformal material.

4. The microwave antenna assembly according to claim 1, further comprising:
a light-emitting device (60) coupled to the filter (58), the light-emitting device (60) adapted to operate when the detection signal is above a predetermined threshold signal,
and/or a speaker (65) coupled to the filter (58), the speaker (65) adapted to operate when the detection signal is above a predetermined threshold signal.

5. The radiation detector according to claim 1, wherein the receiving antenna further comprises:
a third piece of metal (52c) externally attached to the antenna of the microwave antenna assembly (12), wherein the third piece of metal (52c) is coupled to the two pieces of metal (52a, 52b) with a second rectifier (57).

6. The microwave antenna assembly according to claim 1,
wherein the radiation detector (50; 55) is located on the distal end of the microwave antenna.

7. The microwave antenna assembly according to claim 1,
further comprising a gap (51) between the two pieces of metal (52a, 52b) and the gap (51) is located along the antenna at a position of a high RF voltage.

8. The microwave antenna assembly according to claim 1, further comprising a second rectifier (56) coupled between the two pieces of metal (52a, 52b), wherein the first and second rectifiers (54, 56) are oldered to the pieces of metal (52a, 52b) in reverse polarities.

9. The microwave antenna assembly according to claim 1, further comprising:
a hub (22) adapted to couple the microwave antenna assembly (12) to a microwave generator (14);
a radiating section (18) coupled to the hub (22) through the feedline (20);
an inflatable stop (30) surrounding the feedline (20), wherein the inflatable stop (30) is configured to be inflated when the feedline (20) is placed within an abdomen (70) of a patient to prevent the radiating section (18) from inadvertently withdrawing from the abdomen (70).

10. The microwave antenna assembly according to claim 9, wherein the receiving antenna is a ring antenna, and the receiving antenna further comprises:
two dielectric substrates each having a top surface and a bottom surface;
each piece of metal (52a, 52b) is disposed on the top surface of each dielectric substrate; and
a grounding member (G) disposed on the bottom surface of each dielectric substrate, wherein the antenna and grounding members are electrically insulated by the dielectric substrate.
and/or wherein the feedline (20) includes an outer conductor (17) and the receiving antenna is a linear antenna disposed on the outer conductor (17) of the feedline (20) such that a longitudinal axis of the linear antenna is substantially parallel to a longitudinal axis of the feedline (20),
and/or wherein the receiving antenna further comprises a gap (51) between the two pieces of metal (52a, 52b) and the gap (51) is located along the feedline (20) at a position of a high RF voltage,
and/or wherein the receiving antenna is located near a distal end of the inflatable stop.

## Patentansprüche

1. Mikrowellenantennenbaugruppe (12), umfassend eine Mikrowellenantenne, eine koaxiale Speiseleitung (20) und einen Strahlungsdetektor (50, 55), die entlang der koaxialen Speiseleitung (20) angeordnet und konfiguriert sind, um innerhalb der Bauchdecke positioniert zu sein;
wobei der Strahlungsdetektor (50; 55) Folgendes umfasst:
eine Empfangsantenne, die angepasst ist, um Mikrowellenenergie zu empfangen, wobei die Empfangsantenne aus zwei Metallstücken (52a, 52b) gebildet ist, die extern an der Mikrowellenantenne angebracht sind;
mindestens einen Gleichrichter (54, 56), der zwischen die Metallstücke (52a, 52b) gekoppelt ist, der angepasst ist, um mindestens einen Teil der Mikrowellenenergie gleichzurichten; und
einen Filter (58), der mit dem mindestens einen Gleichrichter (54, 56) gekoppelt ist und angepasst ist, um die gleichgerichtete Mikrowellenenergie in ein Detektionssignal umzuwandeln,
wobei jedes der zwei Metallstücke (52a, 52b) eine Geometrie eines Ringes aufweist, der um die koaxiale Speiseleitung (20) gewickelt ist und zur Einführung in ein Abdomen oder eine Körperhöhle konfiguriert ist, wobei jeder Ring eine Breite zwischen 2,54 mm (0,10 Zoll) und 63,5 mm (2,5 Zoll) und eine Dicke zwischen 0,0254 mm (0,001 Zoll) und 0,254 mm (0,010 Zoll) aufweist.

2. Mikrowellenantennenbaugruppe nach Anspruch 1, wobei die Empfangsantenne weiter Folgendes umfasst:
zwei dielektrische Substrate, die jeweils eine Oberseite und eine Unterseite aufweisen;
jedes Metallstück (52a, 52b) ist auf der Oberseite jedes dielektrischen Substrats angeordnet; und
ein Erdungselement, das auf der Unterseite jedes dielektrischen Substrats angeordnet ist, wobei die Antenne und die Erdungselemente durch das dielektrische Substrat elektrisch isoliert sind.

3. Mikrowellenantennenbaugruppe nach Anspruch 2, wobei das dielektrische Substrat aus einem nichtleitenden, konformen Material gebildet ist und die Erdungselemente aus einem leitfähigen, konformen Material gebildet sind.

4. Mikrowellenantennenbaugruppe nach Anspruch 1, weiter umfassend:
eine Licht emittierende Vorrichtung (60), die mit dem Filter (58) gekoppelt ist, wobei die Licht emittierende Vorrichtung (60) angepasst ist, um zu arbeiten, wenn das Detektionssignal über einem vorbestimmten Schwellensignal liegt,
und/oder einen Lautsprecher (65), der mit dem Filter (58) gekoppelt ist, wobei der Lautsprecher (65) angepasst ist, um zu arbeiten, wenn das Detektionssignal über einem vorbestimmten Schwellensignal liegt.

5. Strahlungsdetektor nach Anspruch 1, wobei die Empfangsantenne weiter Folgendes umfasst:
ein drittes Metallstück (52c), das extern an der Antenne der Mikrowellenantennenbaugruppe (12) angebracht ist, wobei das dritte Metallstück (52c) mit den zwei Metallstücken (52a, 52b) mit einem zweiten Gleichrichter (57) gekoppelt ist.

6. Mikrowellenantennenbaugruppe nach Anspruch 1, wobei sich der Strahlungsdetektor (50; 55) am distalen Ende der Mikrowellenantenne befindet.

7. Mikrowellenantennenbaugruppe nach Anspruch 1, weiter einen Spalt (51) zwischen den zwei Metallstücken (52a, 52b) umfassend, und wobei der Spalt (51) sich entlang der Antenne an einer Position einer hohen HF-Spannung befindet.

8. Mikrowellenantennenbaugruppe nach Anspruch 1, weiter einen zweiten Gleichrichter (56) umfassend, der zwischen die zwei Metallstücke (52a, 52b) gekoppelt ist, wobei der erste und der zweite Gleichrichter (54, 56) mit den Metallstücken (52a, 52b) in umgekehrten Polaritäten geordnet sind.

9. Mikrowellenantennenbaugruppe nach Anspruch 1, weiter umfassend:
einen Mittelpunkt (22), der angepasst ist, um die Mikrowellenantennenbaugruppe (12) an einen Mikrowellengenerator (14) zu koppeln;
einen Strahlungsabschnitt (18), der über die Speiseleitung (20) mit dem Mittelpunkt (22) gekoppelt ist;
einen aufblasbaren Anschlag (30), der die Speiseleitung (20) umgibt, wobei der aufblasbare Anschlag (30) so konfiguriert ist, dass er aufgeblasen wird, wenn die Speiseleitung (20) in einem Abdomen (70) eines Patienten angeordnet ist, um zu verhindern, dass sich der Strahlungsabschnitt (18) versehentlich aus dem Abdomen (70) zurückzieht.

10. Mikrowellenantennenbaugruppe nach Anspruch 9, wobei die Empfangsantenne eine Ringantenne ist und die Empfangsantenne weiter Folgendes umfasst:
zwei dielektrische Substrate, die jeweils eine Oberseite und eine Unterseite aufweisen;
jedes Metallstück (52a, 52b) ist auf der Oberseite jedes dielektrischen Substrats angeordnet; und
ein Erdungselement (G), das auf der Unterseite jedes dielektrischen Substrats angeordnet ist, wobei die Antenne und die Erdungselemente durch das dielektrische Substrat elektrisch isoliert sind,
und/oder wobei die Speiseleitung (20) einen Außenleiter (17) einschließt und die Empfangsantenne eine an dem Außenleiter (17) der Speiseleitung (20) angeordnete lineare Antenne ist, sodass eine Längsachse der linearen Antenne im Wesentlichen parallel zu einer Längsachse der Speiseleitung (20) ist,
und/oder wobei die Empfangsantenne weiter einen Spalt (51) zwischen den zwei Metallstücken (52a, 52b) umfasst und der Spalt (51) sich entlang der Speiseleitung (20) an einer Position einer hohen HF-Spannung befindet,
und/oder wobei sich die Empfangsantenne nahe einem distalen Ende des aufblasbaren Anschlags befindet.

## Revendications

1. Ensemble formant antenne à micro-ondes (12), comprenant une antenne à micro-ondes, une ligne d'alimentation coaxiale (20) et un détecteur de rayonnement (50, 55), disposé le long de la ligne d'alimentation coaxiale (20) et configuré pour être positionné à l'intérieur de la paroi abdominale ;
le détecteur de rayonnement (50 ; 55) comprenant :
une antenne de réception conçue pour recevoir de l'énergie micro-ondes, dans laquelle l'antenne de réception est formée à partir de deux pièces de métal (52a, 52b) extérieurement attachées à l'antenne à micro-ondes ;
au moins un redresseur (54, 56) couplé entre les pièces de métal (52a, 52b) conçu pour redresser au moins une portion de l'énergie micro-ondes ; et
un filtre (58) couplé à l'au moins un redresseur (54, 56) et conçu pour transformer l'énergie micro-ondes redressée en un signal de détection,
dans lequel chacune des deux pièces de métal (52a, 52b) a une géométrie d'un anneau enroulé autour de la ligne d'alimentation coaxiale (20) et est configurée pour être insérée dans un abdomen ou une cavité corporelle, chaque anneau ayant une largeur entre 2,54 mm (0,10 pouce) et 63,5 mm (2,5 pouces) et une épaisseur entre 0,0254 mm (0,001 pouce) et 0,254 mm (0,010 pouce).

2. Ensemble formant antenne à micro-ondes selon la revendication 1, dans lequel l'antenne de réception comprend en outre :
deux substrats diélectriques ayant chacun une surface supérieure et une surface inférieure ;
chaque pièce de métal (52a, 52b) est disposée sur la surface supérieure de chaque substrat diélectrique ; et
un élément de mise à la masse disposé sur la surface inférieure de chaque substrat diélectrique, dans lequel les éléments d'antenne et de mise à la masse sont électriquement isolés par le substrat diélectrique.

3. Ensemble formant antenne à micro-ondes selon la revendication 2,
dans lequel le substrat diélectrique est formé à partir d'une matière non conductrice conforme et l'élément de mise à la masse est formé à partir d'une matière conductrice conforme.

4. Ensemble formant antenne à micro-ondes selon la revendication 1, comprenant en outre :
un dispositif électroluminescent (60) couplé au filtre (58), le dispositif électroluminescent (60) étant conçu pour fonctionner lorsque le signal de détection est au-dessus d'un signal de seuil prédéterminé,
et/ou un haut-parleur (65) couplé au filtre (58), le haut-parleur (65) étant conçu pour fonctionner quand le signal de détection est au-dessus d'un signal de seuil prédéterminé.

5. Détecteur de rayonnement selon la revendication 1, dans lequel l'antenne de réception comprend en outre :
une troisième pièce de métal (52c) extérieurement attachée à l'antenne de l'ensemble formant antenne à micro-ondes (12), dans lequel la troisième pièce de métal (52c) est couplée aux deux pièces de métal (52a, 52b) avec un second redresseur (57).

6. Ensemble formant antenne à micro-ondes selon la revendication 1,
dans lequel le détecteur de rayonnement (50 ; 55) est situé sur l'extrémité distale de l'antenne à micro-ondes.

7. Ensemble formant antenne à micro-ondes selon la revendication 1,
comprenant en outre un espacement (51) entre les deux pièces de métal (52a, 52b) et l'espacement (51) est situé le long de l'antenne à une position d'une tension RF élevée.

8. Ensemble formant antenne à micro-ondes selon la revendication 1, comprenant en outre un second redresseur (56) couplé entre les deux pièces de métal (52a, 52b), dans lequel les premier et second redresseurs (54, 56) sont soudés aux pièces de métal (52a, 52b) dans des polarités inverses.

9. Ensemble formant antenne à micro-ondes selon la revendication 1, comprenant en outre :
un concentrateur (22) conçu pour coupler l'ensemble formant antenne à micro-ondes (12) à un générateur de micro-ondes (14) ;
une section de rayonnement (18) couplée au concentrateur (22) par l'intermédiaire de la ligne d'alimentation (20) ;
une butée gonflable (30) entourant la ligne d'alimentation (20), dans laquelle la butée gonflable (30) est configurée pour être gonflée lorsque la ligne d'alimentation (20) est placée à l'intérieur d'un abdomen (70) d'un patient pour empêcher la section de rayonnement (18) de se retirer par mégarde de l'abdomen (70).

10. Ensemble formant antenne à micro-ondes selon la revendication 9, dans lequel l'antenne de réception est une antenne annulaire et l'antenne de réception comprend en outre :
deux substrats diélectriques ayant chacun une surface supérieure et une surface inférieure ;
chaque pièce de métal (52a, 52b) est disposée sur la surface supérieure de chaque substrat diélectrique ; et
un élément de mise à la masse (G) disposé sur la surface inférieure de chaque substrat diélectrique, dans lequel les éléments d'antenne et de mise à la masse sont électriquement isolés par le substrat diélectrique,
et/ou dans lequel la ligne d'alimentation (20) inclut un conducteur extérieur (17) et l'antenne de réception est une antenne linéaire disposée sur le conducteur extérieur (17) de la ligne d'alimentation (20) de sorte qu'un axe longitudinal de l'antenne linéaire est sensiblement parallèle à un axe longitudinal de la ligne d'alimentation (20),
et/ou dans lequel l'antenne de réception comprend en outre un espacement (51) entre les deux pièces de métal (52a, 52b) et l'espacement (51) est situé le long de la ligne d'alimentation (20) à une position d'une tension RF élevée,
et/ou dans lequel l'antenne de réception est située près d'une extrémité distale de la butée gonflable.
